# EUROPEAN PATENT SPECIFICATION

(11) **EP 2 908 871 B1**
(45) Date of publication and mention of the grant of the patent: **08.08.2018**
(21) Application number: 13847381.4
(22) Date of filing: 18.10.2013
(51) Int. Cl.: A61L 15/42, A61K 35/14, C12N 5/00, A61P 29/00, A61L 31/16, A61K 35/19, A61K 35/28

(54) **TECHNIQUES FOR TREATMENT OF ABSCESSES**
VERFAHREN ZUR BEHANDLUNG VON ABSZESSEN
TECHNIQUES DE TRAITEMENT DES ABCÈS

(30) Priority: 18.10.2012 US 201261715506 P
(43) Date of publication of application: 26.08.2015
(73) Proprietor: MAYO FOUNDATION FOR MEDICAL EDUCATION AND RESEARCH, Rochester, MN 55905 (US)
(72) Inventor: DIETZ, Allan B., Chatfield, Minnesota 55923 (US); DOZOIS, Eric J., Rochester, Minnesota 55905 (US); FAUBION, William A., Rochester, Minnesota 55902-6334 (US)
(74) Representative: Kunz, Herbert
(86) International application number: PCT/US2013/065631
(87) International publication number: WO 2014/063022

(56) References cited:
- WO-A2-2008/118913
- RU-C2- 2 435 846
- US-A1- 2008 004 657
- US-A1- 2008 004 657

## Description

### TECHNICAL FIELD

This document relates generally to medical devices, and particularly to medical device systems for treating abscess cavities including fistula.

### BACKGROUND

Unresolved healing is a significant issue in medicine. Failure to heal can lead to ulcers (wounds open to the environment) and abscesses. Abscesses are infected anatomical cavities. A fistula is a type of abscess cavity characterized by a tunnel running between two hollow organs, or between a hollow organ and the surface of the skin. For example, anal fistulae are infected tunnels that develop between the rectum and the skin around the anus. Some anal fistulae are the result of an infection in an anal gland that spreads to the skin. Inflammatory bowel diseases, such as Crohn's disease, also substantially contribute to the formation of fistulae involving the digestive tract. Treatment modalities for anal fistulae depend on the fistula's location and complexity. The general goals of fistulae treatments are to achieve complete fistula closure, to prevent recurrence, and to avoid damaging the sphincter muscles which can lead to fecal incontinence. Healing abscessed cavities is a significant challenge. Likewise, ulcers with their access to the environment, also demonstrate significant challenges to the resolution of disease. US 2008/004657 A discloses a medical graft for the treatment of a fistula, comprising an elongate graft body of a remodelable matrix material, said elongate graft body including at least one conical longitudinal segment of rolled sheet-form extracellular matrix material.

WO 2008/118913 A discloses a composition for use as a soft tissue or void filler, e.g. for treatment of fistulae, comprising fibrinogen, thrombin, at least one plasticizer, microparticles and optionally active agents.

### SUMMARY

This document provides medical device systems for treating fistula and other types of abscess cavities.

The scope of the invention is defined by the claims. Any references in the description to methods of treatment refer to the compounds, pharmaceutical compositions and medicaments of the present invention for use in a method for treatment of the human (or animal) body by therapy (or for diagnosis). The treatment includes filling the abscess cavity or covering the open wound with biocompatible filler capable of conforming to complex shapes while firmly occupying the cavity or covering the wound. In addition, therapeutic agents such as mesenchymal stem cells, with their potent immunoregulatory and reparative properties, can be added to the biocompatible filler to promote healing of the abscess cavity. In some embodiments, the biocompatible filler material can be a solid matrix scaffold, such as a plug that occupies the cavity. In other embodiments, the biocompatible filler can be a fluidic or gel-type biomaterial matrix suitable for injection into the cavity. In some embodiments, the injected fluidic or gel-type biomaterial with therapeutic agents can solidify in situ.

In general, one aspect of this document features methods for treating an abscess cavity of a mammal. The method includes obtaining a biocompatible filler material that is configured for implantation into the abscess cavity, treating the biocompatible filler material with a therapeutic agent wherein the treatment impregnates the biocompatible filler material with the therapeutic agent and wherein the therapeutic agent can promote healing of the abscess cavity, and implanting the treated biocompatible filler material into the abscess cavity.

In another aspect, this document features a method for treating an abscess cavity of a mammal. The method includes providing a biocompatible filler material that is configured for implantation into the abscess cavity wherein the filler material comprises a therapeutic agent that can promote healing of the abscess cavity, and implanting the treated biocompatible filler material into the abscess cavity.

In another aspect, this document features a medical device system for treating an abscess cavity of a mammal. The device system includes a solid matrix biocompatible filler material configured for implantation into the abscess cavity, and a therapeutic agent, wherein the therapeutic agent is bound to the solid matrix biocompatible filler material.

The foregoing and other embodiments can each optionally include one or more of the following features, alone or in combination. For instance, wherein said mammal is a human; wherein said biocompatible filler material is a solid matrix material; wherein said biocompatible filler material is a fluidic or gel-type matrix material; wherein said fluidic or gel-type matrix material is configured to solidify within the abscess cavity; wherein said abscess cavity is a fistula; wherein said therapeutic agent is a stem cell material; and wherein said therapeutic agent is a platelet derivative material.

Particular embodiments of the subject matter described in this specification can be implemented so as to realize one or more of the following advantages. In some embodiments, the addition of therapeutic agents to solid matrix or fluidic matrix scaffolds for implantation into abscess cavities can promote regenerative cell growth and healing. In some cases, the therapeutic agents can improve the success rate of abscess cavity treatments, and promote a faster healing process. Such techniques for treating abscess cavities can provide a less invasive treatment approach in comparison to some traditional surgical treatments. Such techniques can also provide treatments that have a lower potential for adverse events in comparison to some traditional surgical treatments. Such adverse events can include destruction of adjacent tissue, loss of continence, or permanent ileostomy or colostomy. By avoiding adverse events through minimally invasive approaches, patient quality of life can be improved and the cost of care can be reduced. A fluidic matrix may advantageously completely fill complex spaces and conform to the unhealed area so as to inhibit abscess recurrence and promote healing. Additionally, the space occupied by ulcers can be filled such that a barrier exists between unhealed tissue and the surrounding environment to thereby protect from environmental insult, prevent infection, and promote healing.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### BRIEF DESCRIPTION OF THE DRAWINGS

FIG. 1 is an anatomical schematic depicting various types of anal fistula.
FIG. 2 is an illustration of an example solid matrix scaffold filler device for treatment of fistulae.
FIG. 3 is a flowchart of an example method of treating an abscess cavity using a solid matrix scaffold filler device impregnated with a therapeutic agent.
FIG. 4 is a flowchart of an example method of treating an abscess cavity using a fluidic or gel-type biomaterial matrix and/or a therapeutic agent.

Like reference numbers and designations in the various drawings indicate like elements.

### DETAILED DESCRIPTION

This document provides devices, systems, and methods for promoting the healing of abscess cavities. One category of abscess cavities is fistulae. A fistula is a tunnel between two hollow organs, or between a hollow organ and the surface of the skin. Anal fistulae are a particular type of fistula within the fistulae category. This document uses as an example the treatment of anal fistulae to describe the devices, systems, and methods that are provided herein for treating abscess cavities in general. It should be understood, that variations on the example devices, systems, and methods that are described in the context of anal fistula treatments and that are applicable to the treatment of other types of abscess cavities, are within the scope of this document.

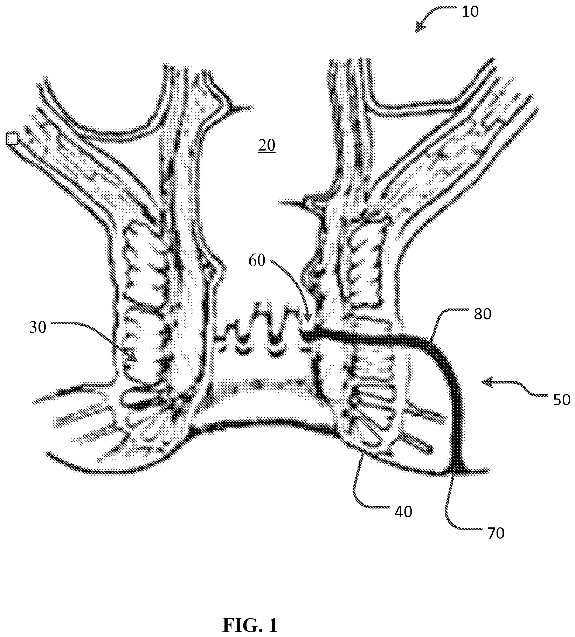

FIG. 1 provides an anatomical schematic drawing of a human's lower colon area 10. Lower colon area 10 includes rectum 20, anal sphincter muscles 30, and skin surface 40. An anal fistula 50 is also depicted. Types of anal fistulae are classified based on the path of their tracts and how close they are to the sphincter muscles. For example, anal fistula 50 is a trans-sphinteric fistula. However, the example devices, systems, and methods provided herein can be applicable to other types of anal fistulae, and to abscess cavities in a broader sense. Anal fistula 50 includes an internal opening 60 (in rectum 20), an external opening 70 (on skin surface 40), and a fistula tract 80. Fistula tract 80 is a tunnel connecting internal opening 60 to external opening 70. Fistula tract 80 is an example of a type of abscess cavity. Fistula tract 80 can be treated by the devices, systems, and methods provided herein. Other types of abscess cavities can be similarly treated.

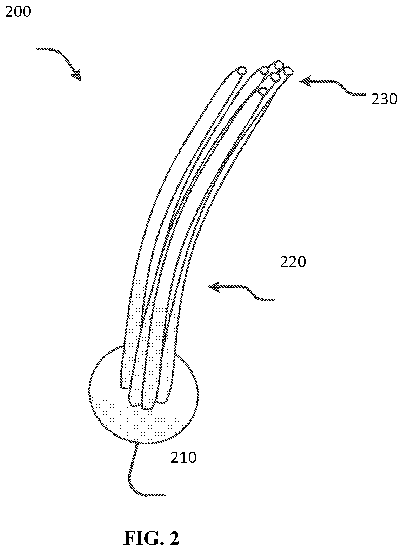

FIG. 2 depicts an example embodiment of a fistula plug device 200 for treating an anal fistula, such as anal fistula 50 of FIG. 1. Fistula plug device 200 is an example of an implantable bioabsorbable device that provides a solid matrix scaffold to support tissue growth. Devices, such as fistula plug device 200 with a solid matrix scaffold, can be implanted into abscess cavities to facilitate tissue regeneration and healing of the cavity. For example, cells can migrate into the solid matrix scaffold, and tissue can be generated as the body gradually absorbs the solid matrix scaffold material. As described further below, solid matrix scaffold devices, such as example fistula plug device 200, can be impregnated with therapeutic agents to enhance their tissue regeneration properties, and to improve the healing of the fistula and other type of abscess cavities. The therapeutic agents can be bound to the scaffolds via any appropriate covalent or non-covalent binding mechanism.

In some embodiments, the solid matrix scaffold device (of which fistula plug device 200 is an example) can be made from synthetic materials. For example, in some embodiments a synthetic bioabsorbable material comprising a non-woven web with open interconnected pores can comprise the solid matrix scaffold device.

In other embodiments, the solid matrix scaffold can comprise an acellular dermal matrix (ADM) material. ADM can be used as a soft tissue replacement. In general, ADM materials are tissue products of selectively preserved extracellular protein in a matrix that is devoid of certain viable cells that would be recognized as foreign by the recipient. ADM material can be converted into various physical configurations for implantation into abscess cavities, such as single strands, multiple strands, intertwined strands, cylindrical shapes, webs, and so on.

Three-dimensional (3D) printers can also be used to print custom-shaped scaffolds. For example, imaging modalities (e.g. CT, MRI, etc.) can be used to create a digital image of an individual patient's fistula tract, which can be used to custom print a 3D scaffold to match the shape of the fistula tract. The solid matrix scaffold materials used for printing can include platelet derivatives, glues or other binder materials, therapeutic agents, scaffold materials, and the like.

By impregnating therapeutic agents into a solid matrix scaffold, such as those solid matrix scaffolds provided by non-woven synthetics or ADM materials, the regenerative and healing process attributable to the solid matrix scaffold can be enhanced. Prior to implantation, a solid matrix scaffold such as fistula plug device 200 can be impregnated with a therapeutic agent to enhance the healing properties of the solid matrix scaffold. For example, fistula plug device 200 can be exposed to a treatment process to bind a therapeutic agent to the synthetic non-woven material of example fistula plug device 200.

Various types of therapeutic agents can be used to impregnate the solid matrix scaffold material and promote tissue regeneration. Such therapeutic agents, in general, are immunoregulatory and reparative agents. For example, in some embodiments, mesenchymal stromal cells (MSC) can be used as a therapeutic agent. In some such embodiments, the MSC can be adipose-derived, autologous MSC. Alternately, in other embodiments autologous MSC derived from other sources can be used, and an allogeneic or xenogeneic source of therapeutics agents may also be used. *See e.g.,* Crespo-Diaz R, et al., Platelet lysate consisting of a natural repair proteome supports human mesenchymal stem cell proliferation and chromosomal stability, Cell Transplantation, 2011; 20(6):797-811, PMID: 21092406.

In another embodiment of a therapeutic agent, expanded adipose derived stem cells (ASC) can be used as a therapeutic agent. An ASC matrix can provide a scaffold upon and within which the patient's own cells can repopulate and revascularize the abscess cavity.

Pooled human platelet derivatives (PL) are yet another example of a therapeutic agent that can be used in further embodiments. PL is a hemoderivate containing a plethora of growth-promoting factors.

Therapeutic agents can also include small molecule drugs and biologics (e.g., growth factors) that facilitate treatment of an abscess cavity.

These and other therapeutic agents, or a combination thereof, can be bound to a solid matrix scaffold, such as fistula plug device 200, in preparation for implantation of the solid matrix scaffold into an abscess cavity such as a fistula. Such a binding of therapeutic agents to a solid matrix scaffold can enhance the tissue regeneration properties of a solid matrix scaffold, to thereby improve the healing of abscess cavities such as fistulae.

The process of binding therapeutic agents to a solid matrix scaffold can be performed, in some embodiments, by suspending the therapeutic agents in various types of solutions or materials that can then be combined with the solid matrix scaffold material to imbibe the scaffold material with the therapeutic agent. In an example embodiment, fistula plug device 200 can be soaked in a solution containing MSC in suspension. Such a process can perform the binding of MSC to the open-celled construction of the example fistula plug device 200. After soaking, in some embodiments that do not employ living cells, the solid matrix scaffold impregnated with a therapeutic agent can be allowed to air-cure to increase the concentration of the therapeutic agent and increase the bond-strength of the therapeutic agent to the material of solid matrix scaffold. Compositions containing the therapeutic agent may also, in some embodiments, be embedded into the solid matrix scaffold by other processing techniques such as spraying, painting, imprinting, and so on. The binding process can also be enhanced by modifying the therapeutic agents and/or surfaces of the solid matrix scaffold to facilitate binding, e.g., with capture antibodies, chemical modification, and so on. With the therapeutic agent thusly impregnated into the solid matrix scaffold device, the device can then be used for implantation into the abscess cavity requiring treatment. For example, example fistula plug device 200 having been impregnated with MSC can be used for implantation into a fistula such as anal fistula 50 of FIG. 1.

Still referring to FIG. 2, in general example fistula plug device 200 includes a disk portion 210 and multiple legs 220 attached to disk portion 210 on their proximal ends, while distal ends 230 can be unattached and individually free. The multiple legs 220 can provide a fistula plug device 200 that is customizable to fit various sizes of fistula tracts. That is, one or more of multiple legs 220 can be trimmed from the disk portion 210 in order to reduce the cross-sectional size of fistula plug device 200 to correlate with the size of the particular fistula tract being treated.

In some embodiments, the fistula plug device can have a variable profile along the length of the device. In general, the fistula plug device can be shaped to fill the cavity and to remain securely implanted. The fistula plug devices, as described above, can be made from synthetic or biogenic materials, and from a composite construction of such materials.

The example fistula plug device 200 with embedded therapeutic agents can be implanted in the tract of a fistula according to the following general exemplary process. First, distal ends 230 can be sutured together. A suitable pulling device can be inserted all the way through fistula tract 80 (refer also to FIG. 1). The pulling device can be a suture, guidewire, hemostat, and the like, in accordance with the particular anatomy and type of the fistula being treated. The end of the pulling device at internal opening 60 can be attached to distal ends 230 of fistula plug device 200. For example, in the case of a suture pulling device, the suture pulling device can be stitched and/or tied to distal ends 230. Or, in the case of a hemostat pulling device, the hemostat can be clamped to distal ends 230. Next, the other end of the pulling device at external opening 70 can be carefully pulled to draw distal ends 230 towards internal opening 60. As distal ends 230 approach internal opening 60, distal ends 230 can be carefully guided into fistula tract 80 through internal opening 60. Fistula plug device 200 can be pulled all the way into fistula 50 until disk portion 210 is flush with internal opening 60. Disk portion 210 can then be sutured or clamped to secure it in place at internal opening 60. If distal ends 230 are protruding from external opening 70, they can be trimmed flush to skin surface 40.

The implanted fistula plug device 200 impregnated with therapeutic agents can provide a scaffold for soft tissue repair to thereby facilitate healing and closure of the fistula. The addition of the therapeutic agent to fistula plug 200 can enhance the speed and success rate of the healing process by providing supplemental immunoregulatory and reparative agents along with the solid matrix scaffold of fistula plug device 200.

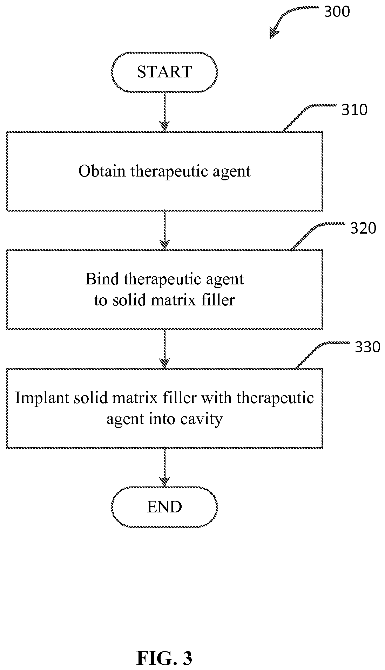

FIG. 3 is a flowchart depicting an example process 300 for treating an abscess cavity using a system including a solid matrix scaffold containing a therapeutic agent. In general, the technique of example process 300 includes filling an abscess cavity with a material that contains a therapeutic agent that promotes tissue growth.

At step 310 a therapeutic agent is obtained. The therapeutic agents can include, for example, MSC, ASC, PL, and the like, as described above. In some cases, the therapeutic agents can be autologous, i.e., derived from the patient to be treated with the therapeutic agent. In some embodiments, MSC and ASC can be, for example, adipose tissue-derived cells. Such agents can, in some cases, require culturing and processing according to established protocols for providing control of the process. For example, MSC for clinical use requires ex vivo expansion of MSC in media containing supplements such as fetal bovine serum or, alternatively, human platelet derivatives (PL). PL is a derivative of human platelets that have been established as a safe and efficient MSC culture supplement for robust MSC cultivation. Further, PL itself can also be used as a therapeutic agent. At step 310, the technique for processing and culturing the therapeutic agent can be performed, or the therapeutic agents can otherwise be obtained.

At step 320, the therapeutic agent obtained at step 310 can be bound to a solid matrix scaffold that will be used later as filler for the abscess cavity. As described above, in some embodiments, the binding process can be performed by soaking the solid matrix scaffold material in a solution containing therapeutic agents in suspension. A solid matrix scaffold comprising an open-matrix material can enhance the bond strength of the therapeutic agents to the solid matrix scaffold. Other processes for imbibing to bind the therapeutic agent to the solid matrix scaffold can also be used, e.g., spraying, painting, absorbing, and so on.

In some cases, a solution for imbibing the solid matrix scaffold with one or more therapeutic agents can be designed to include (in addition to the one or more therapeutic agents described herein) components including, without limitation, salts, buffers, growth factors, cell signaling agents, or small molecule modulators. In these cases, a solid matrix scaffold material can be soaked in the solution, or imbibed with the solution using another suitable technique.

In one example, a solid matrix scaffold material can be soaked in a solution (e.g., a PL-containing solution) for a range of time from about 3 minutes to about 5 days (e.g., from about 5 minutes to about 5 days, from about 15 minutes to about 5 days, from about 1 hour to about 5 days, from about 3 hours to about 5 days, from about 6 hours to about 5 days, from about 18 hours to about 5 days, from about 1 day to about 5 days, from about 2 days to about 5 days, from about 3 days to about 5 days, or from about 4 days to about 5 days). In another example, a solid matrix scaffold material can be soaked in a solution (e.g., a PL-containing solution) for a range of time from about 3 minutes to about 4 days (e.g., from about 3 minutes to about 3 days, from about 3 minutes to about 2 days, from about 3 minutes to about 1 day, from about 3 minutes to about 12 hours, from about 3 minutes to about 6 hours, from about 3 minutes to about 4 hours, or from about 3 minutes to about 2 hours). In another example, a solid matrix scaffold material can be soaked in a solution (e.g., a PL-containing solution) for a range of time from about 1 hour to about 3 days (e.g., from about 2 hours to about 2 days, from about 2 hours to about 1 day, or from about 1 day to about 3 days).

The soaking step can be performed at any appropriate temperature. In one example, the soaking step can be performed at a range of temperatures from about 2 °C to about 45 °C (e.g., from about 10 °C to about 40 °C, from about 20 °C to about 37 °C, or from about 30 °C to about 40 °C). In another example, the soaking step can be performed at a range of temperatures from about 18 °C to about 26 °C (e.g., from about 20 °C to about 24 °C or from about 21 °C to about 23 °C). In another example, the soaking step can be performed at a range of temperatures from about 30 °C to about 44 °C (e.g., from about 33 °C to about 41 °C or from about 36 °C to about 38 °C). In another example, the soaking step can be performed at a range of temperatures from about 1°C to about 7 °C (e.g., from about 3 °C to about 5 °C).

In another example, a solid matrix scaffold material can be soaked in a solution (e.g., a PL-containing solution) for about 24 hours at about 37 °C.

At step 330, the solid matrix scaffold imbibed with a therapeutic agent is implanted into the abscess cavity being treated. With the system in place in the abscess cavity, the solid matrix scaffold can promote tissue growth and healing of the abscess cavity. With impregnated therapeutic agents included in the solid matrix scaffold, such as those solid matrix scaffolds provided by non-woven synthetics or ADM materials, the regenerative and healing process attributable to the solid matrix scaffold can be enhanced.

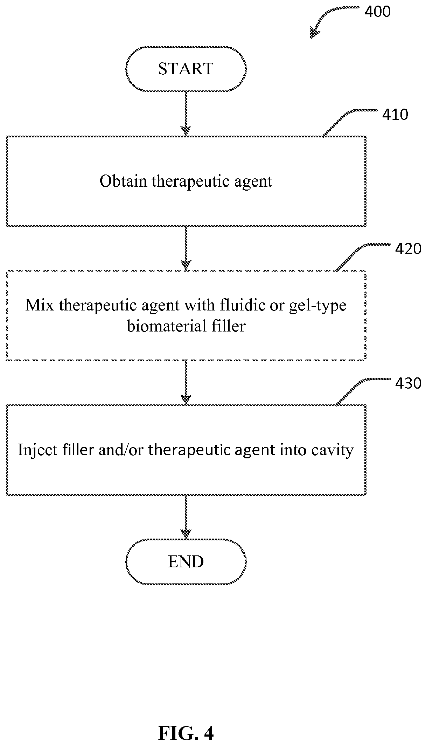

FIG. 4 is a flowchart depicting an example process 400 for treating an abscess cavity using a system including a fluidic or gel-type matrix material containing a therapeutic agent. In general, the technique of example process 400 includes injecting an abscess cavity with a fluidic or gel-type material that contains an agent that promotes tissue growth. The injected material can be precisely conformable to the complex spaces of a wide variety of abscess cavities. In some embodiments, the injected material can solidify in situ to result in a filler that firmly occupies the abscess space and includes a therapeutic agent to promote healing.

At step 410, a therapeutic agent is obtained. The therapeutic agents can include, for example, MSC, ASC, PL, small molecule drugs, biologic agents, and the like, as described above. The processes of obtaining such therapeutic agents are described in reference to step 310 of FIG. 3 above.

At step 420, the therapeutic agent is optionally mixed with a fluidic or gel-type biomaterial matrix material that will be used to act as a filler for the abscess cavity. This step is optional because, for example, in some embodiments the therapeutic material alone can be injected at step 430, without having been mixed with any other fluidic biomaterial. For example, PL can, in some embodiments, be injected into an abscess cavity without being mixed with a fluidic biomaterial. However, in some embodiments the therapeutic material can be delivered to the abscess cavity by mixing it with a fluidic or gel-type biomaterial matrix material that acts as a delivery device for the therapeutic material and a filler for the abscess cavity. In some embodiments, the fluidic or gel-type biomaterial filler can be a glue-based material, such as a fibrous protein two-part fibrin glue. Other fluidic or gel-type biocompatible materials, both glue-based and non-glue-based, that will solidify in situ to form a strong and flexible bond may also be utilized. For example, other usable types of materials can include anoacrylates, collagen-based compounds, glutaraldehyde glues, hydrogels, and purified bovine serum albumin with glutaraldehyde.

At step 430, the fluidic or gel-type biomaterial containing or comprising the therapeutic agent can be injected into the abscess cavity. First, using the example of a fistula tunnel, one of the fistula openings can be sealed shut using sutures or clips. The fluidic or gel-type biomaterial can then be injected into the fistula tunnel through the other opening. The fluidic or gel-type biomaterial can be injected using, for example, a syringe or a catheter, or a combination thereof. In some cases, the materials to be injected can be mixed ex vivo just prior to injection, so as to avoid solidification prior to injection. In other cases, a dual syringe can be used to inject the materials, thereby preventing the mixing and potential solidification of the fluidic or gel-type materials prior to their filling the abscess cavity space. After injection of the fluidic or gel-type biomaterial containing or comprising the therapeutic agent, the material can solidify within the abscess cavity. The solidified material can provide a scaffold that firmly occupies the abscess space to promote tissue growth, and that includes a therapeutic agent to promote healing.

## Claims

1. A medical device system for use in treating an abscess cavity of a mammal, said device system comprising:
(a) a plug comprising a disk portion and multiple legs attached to said disk portion, said disk portion and legs configured for implantation into the abscess cavity, wherein said disk portion and legs are made of solid matrix biocompatible filler material, and
(b) a therapeutic agent, wherein the therapeutic agent is bound to the solid matrix biocompatible filler material.

2. The medical device system of claim 1, wherein said mammal is a human.

3. The medical device system of claim 1 or 2, wherein said abscess cavity is a fistula.

4. The medical device system of any of claims 1-3, wherein said fistula is an anal fistula.

5. The medical device system of any of claims 1-4, wherein said therapeutic agent is a) a stem cell material; or b) a platelet derivative material.

6. The medical device system of claim 5, wherein the stem cell material is adipose derived stem cells and said adipose-derived stem cells are imbibed into said solid matrix biocompatible filler material.

7. A method for obtaining a medical device system for use in treating an abscess cavity of a mammal, said method comprising:
(a) providing a plug comprising a disk portion and multiple legs attached to said disk portion, said disk portion and legs configured for implantation into the abscess cavity, wherein said disk portion and legs are made of solid matrix biocompatible filler material,
(b) treating the solid matrix biocompatible filler material with a therapeutic agent, wherein the treatment impregnates the solid matrix biocompatible filler material with the therapeutic agent, and wherein the therapeutic agent can promote healing of the abscess cavity.

8. The method of claim 7, wherein said mammal is a human.

9. The method of claims 7 or 8, wherein said abscess cavity is a fistula.

10. The method of any of claims 7-9, wherein said fistula is an anal fistula.

11. The method of any of claims 7-10, wherein said therapeutic agent is a) a stem cell material; or b) a platelet derivative material.

12. The method of claim 11, wherein the stem cell material is adipose derived stem cells and said adipose-derived stem cells are imbibed into said solid matrix biocompatible filler material.

## Patentansprüche

1. Medizinproduktsystem zur Verwendung in der Behandlung einer Abszesshöhle eines Säugers, wobei das Produktsystem umfasst:
(a) einen Tamponadepfropf, der einen Scheibenanteil und mehrere an dem Scheibenanteil befestigte Beine umfasst, wobei der Scheibenanteil und die Beine zur Implantation in die Abszesshöhle ausgelegt sind, wobei der Scheibenanteil und die Beine aus biokompatiblem festem Matrixfüllmaterial hergestellt sind; und
(b) ein therapeutisches Mittel, wobei das therapeutische Mittel an das biokompatible feste Matrixfüllmaterial gebunden ist.

2. Medizinproduktsystem nach Anspruch 1, wobei das Säugetier ein Mensch ist.

3. Medizinproduktsystem nach Anspruch 1 oder 2, wobei die Abszesshöhle eine Fistel ist.

4. Medizinproduktsystem nach einem der Ansprüche 1 bis 3, wobei die Fistel eine Analfistel ist.

5. Medizinproduktsystem nach einem der Ansprüche 1 bis 4, wobei das therapeutische Mittel a) Stammzellmaterial oder b) von Thrombozyten abgeleitetes Material ist.

6. Medizinproduktsystem nach Anspruch 5, wobei das Stammzellmaterial von Fettzellen abgeleitete Stammzellen ist und die von Fettzellen abgeleiteten Stammzellen in das biokompatible feste Matrixfüllmaterial aufgenommen worden sind.

7. Verfahren zum Erhalten eines Medizinproduktsystems zur Verwendung in der Behandlung einer Abszesshöhle eines Säugers, wobei das Verfahren umfasst:
(a) Bereitstellen eines Tamponadepfropfs, der einen Scheibenanteil und mehrere an dem Scheibenanteil befestigte Beine umfasst, wobei der Scheibenanteil und die Beine zur Implantation in die Abszesshöhle ausgelegt sind, wobei der Scheibenanteil und die Beine aus biokompatiblem festem Matrixfüllmaterial hergestellt sind;
(b) Behandeln des biokompatiblen festen Matrixfüllmaterials mit einem therapeutischen Mittel, wobei die Behandlung das biokompatible feste Matrixfüllmaterial mit dem therapeutischen Mittel imprägniert, und wobei das therapeutische Mittel Heilung der Abszesshöhle fördern kann.

8. Verfahren nach Anspruch 7, wobei der Säuger ein Mensch ist.

9. Verfahren nach Anspruch 7 oder 8, wobei die Abszesshöhle eine Fistel ist.

10. Verfahren nach einem der Ansprüche 7 bis 9, wobei die Fistel eine Analfistel ist.

11. Verfahren nach einem der Ansprüche 7 bis 10, wobei das therapeutische Mittel a) Stammzellmaterial oder b) von Thrombozyten abgeleitetes Material ist.

12. Verfahren nach Anspruch 11, wobei das Stammzellmaterial von Fettzellen abgeleitete Stammzellen ist und die von Fettzellen abgeleiteten Stammzellen in das biokompatible feste Matrixfüllmaterial aufgenommen worden sind.

## Revendications

1. Système de dispositif médical pour utilisation dans le traitement d'une cavité d'abcès d'un mammifère, ledit système de dispositif comprenant :
(a) une cartouche comprenant une partie de disque et des pattes multiples fixées à ladite partie de disque, lesdites partie de disque et pattes étant configurées pour implantation dans la cavité d'abcès, dans lequel lesdites partie de disque et pattes sont constituées d'un matériau de charge biocompatible à matrice solide ;
(b) un agent thérapeutique, dans lequel l'agent thérapeutique est lié au matériau de charge biocompatible à matrice solide.

2. Système de dispositif médical selon la revendication 1, ledit mammifère étant un humain.

3. Système de dispositif médical selon la revendication 1 ou 2, ladite cavité d'abcès étant une fistule.

4. Système de dispositif médical selon l'une quelconque des revendications 1 à 3, ladite fistule étant une fistule anale.

5. Système de dispositif médical selon l'une quelconque des revendications 1 à 4, dans lequel ledit agent thérapeutique est a) un matériau de cellules souches ; ou b) un matériau dérivé de plaquettes.

6. Système de dispositif médical selon la revendication 5, dans lequel le matériau de cellule souche est constitué de cellules souches dérivées de tissu adipeux et lesdites cellules souches dérivées de tissu adipeux sont imbibées dans ledit matériau de charge biocompatible à matrice solide.

7. Procédé d'obtention d'un système de dispositif médical pour utilisation dans le traitement d'une cavité d'abcès d'un mammifère, ledit procédé comprenant :
(a) la fourniture d'une cartouche comprenant une partie de disque et des pattes multiples fixées à ladite partie de disque, lesdites partie de disque et pattes étant configurées pour implantation dans la cavité d'abcès, dans lequel lesdites partie de disque et pattes sont constituées d'un matériau de charge biocompatible à matrice solide,
(b) le traitement du matériau de charge biocompatible à matrice solide avec un agent thérapeutique, dans lequel le traitement imprègne le matériau de charge biocompatible à matrice solide avec l'agent thérapeutique, et dans lequel l'agent thérapeutique peut favoriser la cicatrisation de la cavité d'abcès.

8. Procédé selon la revendication 7, dans lequel ledit mammifère est un humain.

9. Procédé selon les revendications 7 ou 8, dans lequel ladite cavité d'abcès est une fistule.

10. Procédé selon l'une quelconque des revendications 7 à 9, dans lequel ladite fistule est une fistule anale.

11. Procédé selon l'une quelconque des revendications 7 à 10, dans lequel ledit agent thérapeutique est a) un matériau de cellules souches ; ou b) un matériau dérivé de plaquettes.

12. Procédé selon la revendication 11, dans lequel le matériau de cellules souches est constitué de cellules souches dérivées de tissu adipeux et lesdites cellules souches dérivées de tissu adipeux sont imbibées dans ledit matériau de charge biocompatible à matrice solide.
